# EUROPÄISCHE PATENTSCHRIFT

(11) **EP 1 165 081 B1**
(45) Veröffentlichungstag und Bekanntmachung des Hinweises auf die Patenterteilung: **25.05.2005**
(21) Anmeldenummer: 99973809.9
(22) Anmeldetag: 16.11.1999
(51) Int. Cl.: A61K 31/445, A61K 9/20, A61K 9/50, A61K 9/00

(54) **VERFAHREN ZUM HERSTELLEN TOLPERISON-ENTHALTENDER PHARMAZEUTISCHER ZUBEREITUNGEN ZUR ORALEN VERABREICHUNG**
PROCESS FOR PREPARING TOLPERISON-CONTAINING PHARMACEUTICAL PREPARATIONS FOR ORAL ADMINISTRATION
PROCEDE DE PREPARATION DE COMPOSITIONS PHARMACEUTIQUES DE TOLPERISONE POUR ADMINISTRATION ORALE

(30) Priorität: 01.04.1999 AT 59499
(43) Veröffentlichungstag der Anmeldung: 02.01.2002
(73) Patentinhaber: Sanochemia Pharmazeutika AG, 1091 Wien (AT)
(72) Erfinder: FRANTSITS, Werner, A-1130 Wien (AT)
(74) Vertreter: Beer, Manfred, Dipl.-Ing.
(86) Internationale Anmeldenummer: PCT/AT1999/000276
(87) Internationale Veröffentlichungsnummer: WO 2000/059508

(56) Entgegenhaltungen:
- GB-A- 1 480 175
- US-A- 4 702 918
- US-A- 4 996 058
- DATABASE CHEMABS [Online] CHEMICAL ABSTRACTS SERVICE, COLUMBUS, OHIO, US AKITO, EIICHIRO ET AL: "Stable toluperizone hydrochloride sirup" retrieved from STN Database accession no. 85:182420 CA XP002129514 in der Anmeldung erwähnt & JP 51 091315 A (NIPPON KAYAKU CO., LTD., JAPAN) 10. August 1976 (1976-08-10)
- PATENT ABSTRACTS OF JAPAN vol. 007, no. 248 (C-193), 4. November 1983 (1983-11-04) & JP 58 135806 A (NIPPON KAYAKU KK), 12. August 1983 (1983-08-12)
- DATABASE CHEMABS [Online] CHEMICAL ABSTRACTS SERVICE, COLUMBUS, OHIO, US YOKOYAMA, TERUYOSHI ET AL.: "Determination of tolperisone enantiomers in plasma and their disposition in rats" retrieved from STN Database accession no. 116:187387 XP002129515 & YOKOYAMA, TERUYOSHI ET AL.: " Determination of tolperisone enantiomers in plasma and their disposition in rats" CHEM. PHARM. BULL., Bd. 40, Nr. 1, 1992, Seiten 272-274,
- PATENT ABSTRACTS OF JAPAN vol. 002, no. 081 (C-016), 28. Juni 1978 (1978-06-28) & JP 53 040779 A (NIPPON KAYAKU CO LTD), 13. April 1978 (1978-04-13)

## Beschreibung

Die Erfindung betrifft Verfahren zum Herstellen einer pharmazeutischen Zubereitung zur oralen Verabreichung, die Tolperison oder ein Salz davon enthält, wobei die Zubereitung zur verzögerten Freisetzung des Wirkstoffes Tolperison zubereitet ist.

Tolperison ist der internationale Freiname für das Muskelrelaxans (RS)-2,4'-Dimethyl-3-piperidinopropiophenon) mit der Summenformel C₁₆H₂₃NO.

Tolperison und dessen Salze sind als Wirkstoff zum Verbessern nicht nur verschiedener Symptome im Hinblick auf spastische Paralyse, sondern auch Muskeltonus, der von Krankheiten, wie cervikalem Syndrom, Gelenksentzündung und Rückenschmerzen, herrührt, bekannt.

Tolperison enthaltende Arzneimittel sind in den verschiedenen Zubereitungsformen bekannt. So beschreibt die EP 0 295 411 B eine pharmazeutische Zubereitung zur perkutanen Verabreichung von Tolperison oder einem Salz davon.

Auch andere Darreichungsformen von Tolperison enthaltenden Arzneimitteln sind bekannt. So beschreibt die JP 51091315 A einen stabilen Sirup von Tolperison, der zur oralen Verabreichung bestimmt ist.

Nachteilig beim oralen Verabreichen von Tolperison oder einem Salz davon ist es, daß die Wirkung rasch nachläßt, so daß Tolperison enthaltende Präparate mehrmals täglich genommen werden müssen, und der gastrointestinale Trakt des Patienten geschädigt werden kann.

Nachteil der perkutanen Anwendung, wie sie aus der EP 0 295 411 B bekannt ist, ist die nur unzureichende perkutane Absorption des pharmazeutischen Wirkstoffes Tolperison.

Der Wirkstoff Tolperison liegt als 50/50-Racemat vor. Untersuchungen haben ergeben, daß im Blut zu 90% das (-)-Isomere und zu nur 10% das (+)-Isomere von Tolperison vorliegt. Es wurde bislang nicht abschließend geklärt, ob das im Blut (von Menschen) vorliegende 90/10-Racemat durch eine Umracemisierung oder durch verstärkte Resorption des (-).-Isomeren entsteht.

Die JP 58135806 A beschreibt die Verwendung von Zellulose-Derivaten zum Herstellen mehrlagiger Beschichtungen von pharmazeutischen Zubereitungen. Nach dem in der JP 58135806 A beschriebenen Verfahren soll ein festes Arzneimittel nacheinander mit Hydroxypropylmethylzellulose, Hydroxypropylmethylzellulosephthalat und mit Polyvinylacetaldiethylaminoacetat beschichtet werden. So soll der Geruch/Geschmack von Arzneimitteln wie Tolperison-Hydrochlorid maskiert werden.

Der Erfindung liegt die Aufgabe zugrunde, eine Zubereitung, die Tolperison oder ein Salz davon enthält, zur Verfügung zu stellen, die oral verabreicht werden kann, ohne daß die Nachteile der bekannten oral verabreichbaren Zubereitungen des Tolperison auftreten.

Erfindungsgemäß wird diese Aufgabe mit Verfahren gelöst, welche die Merkmale der unabhängigen Ansprüche aufweisen.

Bevorzugte und vorteilhafte Ausgestaltungen der erfindungsgemäßen Zubereitung sind Gegenstand der abhängigen Unteransprüche.

Durch die erfindungsgemäß erhältliche Zubereitung wird die bei bekannten, oralen Zubereitungen des Tolperison oder dessen Salzen rasch abflauende Wirkung durch eine lang andauernde Wirkung ersetzt, da die Zubereitung so hergestellt ist, daß der Wirkstoff Tolperison oder das Salz davon nur verzögert freigesetzt wird. Insbesondere kann bei der erfindungsgemäßen Zubereitung vorteilhaft sein, daß die Verzögerung der Freisetzung des Wirkstoffes Tolperison so eingestellt ist, daß Tolperison vornehmlich im Darm resorbiert wird.

Die Vorteile der kontrollierten Freisetzung von pharmazeutischen Wirkstoffen sind auf dem Gebiet der Pharmazeutik gut bekannt und bestehen u.a. darin, daß ein gewünschter Gehalt an Wirkstoff im Blut über eine vergleichsweise lange Zeitspanne aufrechtgehalten werden kann, so daß der Patient nicht mehr gezwungen ist, ein Arzneimittel mehrmals täglich zu sich zu nehmen.

Die erfindungsgemäß erhältlichen Zubereitungen mit verzögerter Freisetzung von Tolperison oder Salzen davon können beispielsweise in Kombination mit verschiedenen Hydrogelen, die synthetisch, halbsynthetisch oder natürlichen Ursprungs sein können, vorliegen.

Orale Zubereitungen mit verzögerter Freisetzung des Wirkstoffes - im vorliegenden Tolperison oder ein Salz davon - sollen so einstellbar sein, daß die Freisetzungsraten und -profile den physiologischen und chronotherapeutischen Anforderungen entsprechend eingestellt werden können. Dies erlaubt die erfindungsgemäße Zubereitung. Untersuchungen haben gezheigt, dass das (-)-Isomere und das (+)-Isomere des Tolpersion praktisch gleich wirksam sind. Daher sind auch racemische Mischungen von Isomeren des Tolperison im wesentlichen gleich wirksam wie das eine oder das nadere Isomer alleine.

Tolperison kann in den erfindungsgemäßen Zubereitungen als 50/50-Racemat oder als von dem 50/50-Racemat abweichendes racemisches Gemisch vorliegen. Racemate, in welchen der Gehalt an dem (-)-Isomeren höher ist, als jener des (+)-Isomeren werden ebenfalls in Betracht gezogen. Racemate mit überwiegendem Anteil des (-)-Isomeren von Tolperison (2,4'-Dimethyl-3-piperidinopropiophenon) können als 90/10-Racemate vorliegen.

Nachstehend werden Beispiele für pharmazeutische Zubereitungen, die gemäß der vorliegenden Erfindung erhältlich sind, angegeben.

### Beispiel 1:

Kristallines Tolperison-Hydrochlorid (50/50-Racemat) mit einer Korngröße von 30 und 60 mesh wurden in eine mit Luftstrom betriebene Beschichtungs-Säule eingetragen und mit einem Gemisch aus einer Polymerlösung in Chloroform, die Ethylcellulose und Hydroxipropylcellulose sowie Methanol enthält, beschichtet. Die Beschichtungslösung wurde mit 2,5 bar Druck in die Säule mit einer Geschwindigkeit von 60 ml/min eingesprüht. Die Einlaßtemperatur betrug etwa 60°C. Nachdem das Zuführen der Beschichtung beendet war, wurden die rasch getrockneten, mit der Polymerbeschichtung versehenen Tolperison-Kristalle aus der Beschichtungs-Säule nach unten entnommen.

### Beispiel 2:

In diesem Beispiel wurde eine wässerige, flüssige Suspension von Tolperison (50/50-Racemat) mit verzögerter Freisetzung hergestellt. Das wässerige Vehikel wurde mit Tolperison gesättigt und enthielt in Wasser suspendiert mikroverkapseltes Tolperison. Tolperison ist in der gesättigten, wässerigen Lösung in einer seiner Löslichkeit entsprechenden Menge enthalten. Durch Verabreichung einer Suspension aus Tolperison enthaltenden Mikrokapseln in einem mit Tolperison gesättigten, wässerigen Vehikel ist es möglich, Tolperison in ausreichender Dosis zur Verfügung zu stellen. Dies kann dadurch erreicht werden, daß Tolperison in Form von suspendierten, Tolperison enthaltendenden Mikrokapseln und Tolperison als wässerige Lösung in dem jeweils benötigten Mischungsverhältnis zur Verfügung gestellt werden. Die Menge von Tolperison in den Mikrokapseln kann vergrößert werden, um der Menge der durch die Mikrokapseln ersetzten Lösung von Tolperison Rechnung zu tragen.

### Beispiel 3:

In diesem Beispiel wird zunächst ein Bindemittel für die verzögerte Freisetzung des Wirkstoffes Tolperison hergestellt und dann zu diesem der Wirkstoff Tolperison (50/50-Racemat) zugegeben, worauf schließlich zu Tabletten verpreßt wird. Das Bindemittel für die verzögerte Freisetzung von Tolperison wird hergestellt, indem die entsprechenden Mengen von Xanthan-Gummi, Johannisbrot-Gummi, Calciumsulfat und Dextrose in einem Hochgeschwindigkeitsmischer/ Granulierer 2 min lang trocken vermengt wurden. Noch während des Vermengens wurde dem zunächst noch trockenen Gemenge Wasser zugesetzt und dann 2 min lang granuliert. Die erhaltenen Granülen wurden in einem Fließbetttrockner getrocknet. Die so erhaltenen, getrockneten Granülen wurden dann auf eine Korngröße von 20 mesh gemahlen. Beispielsweise wurde das Bindemittel aus einem Gemenge aus 25 % XanthanGummi, 25 % Johannisbrot-Gummi, 40 % Dextrose, 10 % Kalziumsulfat und 10 % Wasser (zugegeben während des Granulierens) hergestellt.

Als nächstes wurde das Bindemittel für verzögerte Freigabe mit der jeweils gewünschten Menge an Tolperison, das als Hydrochloridsalz eingesetzt wurde, in einem Hochgeschwindigkeitsmischer/Granulierer 2 min lang gemischt. Bei laufendem Mischer wurde dem Gemenge eine Lösung von Äthylcellulose in Äthanol zugesetzt und das Gemenge 2 min lang granuliert. Die erhaltenen Granülen wurden in einem Fließbetttrockner getrocknet und dann auf eine Korngröße von 20 mesh gemahlen. Nach Zugabe eines geeigneten Tablettierhilfsmittels (z.B. Natriumstearylfumarat) wurde weitere 5 min gemischt. Dieses schlußendlich erhaltene Gemenge wurde zu Tabletten gepreßt.

Die so hergestellten Tabletten können in ihrer Freisetzungsrate dadurch beeinflußt werden, daß die Menge von Gummi in den Zubereitungen erhöht wird, da dann die Freisetzung des Wirkstoffes (Tolperison) abnimmt. So ist es beispielsweise möglich, mit den Tabletten gemäß Beispiel 3 eine 24-Stunden-Dosierung von Tolperison zur Verfügung zu stellen.

### Beispiel 4:

Eine Tolperison-Hydrochlorid (50/50-Racemat) als Wirkstoff enthaltende Tablette mit verzögerter Freisetzung des Wirkstoffes enthält
Tolperisonhydrochlorid
Lactose
Methylhydroxypropylcellulose
Farbstoff
Wasser zur Granulatbildung
Magnesiumstearat
feindisperses Siliciumdioxid.

Zur Herstellung der Tabletten wurde wie folgt vorgegangen:

Der Farbstoff wurde in Wasser eingerührt, Tolperisonhydrochlorid, Lactose und Methylhydroxypropylcellulose wurden in einen Wirbelschichtgranulator eingegeben und mit der wässerigen, den Farbstoff enthaltenden Lösung granuliert. Die erhaltenen Granülen wurden zusammen mit Magnesiumstearat und feindispersen Siliziumdioxid durch ein Sieb mit einer Maschenweite von 1,25 mm gearbeitet und in einem Mischer homogenisiert. Das so erhaltene Gemenge wurde auf einer Tablettiermaschine zu Tabletten verpreßt.

### Beispiel 5:

Nach der Vorschrift von Beispiel 1 wurde eine Zubereitung, die ein 90/10-Racemat (mit überwiegendem Anteil an (-)-Isomeren) des Tolperison enthält, hergestellt.

### Beispiel 6:

Nach der Vorschrift von Beispiel 2 wurde eine Zubereitung, die ein 80/20-Racemat (mit überwiegendem Anteil an (-)-Isomeren) des Tolperison enthält, hergestellt.

### Beispiel 7:

Nach der Vorschrift von Beispiel 2 wurde eine Zubereitung, die ein 70/30-Racemat (mit überwiegendem Anteil an (-)-Isomeren) des Tolperison enthält, hergestellt.

### Beispiel 8:

Nach der Vorschrift von Beispiel 3 wurde eine Zubereitung, die ein 90/10-Racemat (mit überwiegendem Anteil an (-)-Isomeren) des Tolperison enthält, hergestellt.

### Beispiel 9:

Nach der Vorschrift von Beispiel 4 wurde eine Zubereitung, die ein 65/35-Racemat (mit überwiegendem Anteil an (-)-Isomeren) des Tolperison enthält, hergestellt.

### Beispiel 10:

Nach der Vorschrift von Beispiel 4 wurde eine Zubereitung, die ein 90/10-Racemat (mit überwiegendem Anteil an (-)-Isomeren) des Tolperison enthält, hergestellt.

Zusammenfassend kann ein Ausführungsbeispiel der Erfindung wie folgt beschrieben werden:

Eine pharmazeutische Zubereitung enthält Tolperison oder ein Salz davon als Wirkstoff in Form eines racemischen Gemisches, das ein 50/50-Racemat oder ein Racemat mit überwiegendem Anteil des (-)-Isomeren von Tolperison sein kann. Die zur oralen Verabreichung, bestimmte pharmazeutische Zubereitung wird als festes oder flüssiges, oral verabreichbares Arzneimittel formuliert, wobei der Wirkstoff Tolperison, der als 50/50-Racemat oder als Racemat mit überwiegendem Anteil des (-)-Isomeren oder des (+)-Isomeren vorliegt, aus der Zubereitung im menschlichen Körper verzögert, bevorzugt im Darmtrakt freigesetzt wird.

## Patentansprüche

1. Verfahren zum Herstellen einer pharmazeutischen Zubereitung zur oralen Verabreichung, die Tolperison oder ein Salz davon enthält, wobei die Zubereitung zur verzögerten Freisetzung des Wirkstoffes Tolperison zubereitet ist, **dadurch gekennzeichnet, dass** kristallines Tolperison-Hydrochlorid als 50/50-Racemat in einer mit Luftstrom betriebenen Beschichtungs-Säule mit einem Gemisch aus Methanol und einer Lösung von Ethylcellulose und Hydroxipropylcellulose in Chloroform beschichtet wird, indem die Lösung bei einer Temperatur von 60°C am Einlass in die Beschichtungssäule mit 2,5 bar Druck und mit einer Geschwindigkeit von 60 ml/min eingesprüht wird und dass die mit der Polymerbeschichtung versehenen Tolperisonkristalle getrocknet werden.

2. Verfahren zum Herstellen einer pharmazeutischen Zubereitung zur oralen Verabreichung, die Tolperison oder ein Salz davon enthält, wobei die Zubereitung zur verzögerten Freisetzung des Wirkstoffes Tolperison zubereitet ist, **dadurch gekennzeichnet, dass** Tolperison als 90/10-Racemat mit überwiegendem Anteil an (-)-Isomeren in einer mit Luftstrom betriebenen Beschichtungs-Säule mit einem Gemisch aus Methanol und einer Lösung von Ethylcellulose und Hydroxipropylcellulose in Chloroform beschichtet wird, indem die Lösung bei einer Temperatur von 60°C am Einlass in die Beschichtungssäule mit 2,5 bar Druck und mit einer Geschwindigkeit von 60 ml/min eingesprüht wird und dass die mit der Polymerbeschichtung versehenen Tolperisonkristalle getrocknet werden.

3. Verfahren zum Herstellen einer pharmazeutischen Zubereitung zur oralen Verabreichung, die Tolperison oder ein Salz davon enthält, wobei die Zubereitung zur verzögerten Freisetzung des Wirkstoffes Tolperison zubereitet ist, **dadurch gekennzeichnet, dass** in einer wässerigen Lösung von Tolperison, die mit Tolperison gesättigt ist, Mikrokapseln, die Tolperison enthalten, suspendiert werden.

4. Verfahren nach Anspruch 3, **dadurch gekennzeichnet, dass** das gelöste und das in den Mikrokapseln enthaltene Tolperison als 50/50-Racemat vorliegt.

5. Verfahren nach Anspruch 3, **dadurch gekennzeichnet, dass** das gelöste und das in den Mikrokapseln enthaltene Tolperison als 80/20-Racemat mit überwiegendem Anteil an (-)-Isomeren vorliegt.

6. Verfahren nach Anspruch 3, **dadurch gekennzeichnet, dass** das gelöste und das in Mikrokapseln enthaltene Tolperison als 70/30-Racemat mit überwiegendem Anteil an (-)-Isomeren vorliegt.

7. Verfahren zum Herstellen einer pharmazeutischen Zubereitung zur oralen Verabreichung, die Tolperison enthält, wobei die Zubereitung zur verzögerten Freisetzung des Wirkstoffes Tolperison zubereitet ist, **dadurch gekennzeichnet, dass** ein Bindemittel hergestellt wird, indem Xanthan-Gummi, Johannisbrot-Gummi, Calciumsulfat und Dextrose in einem Hochgeschwindigkeitsmischer/Granulierer 2 min lang trocken vermengt werden, dass nach Zugabe von Wasser 2 min lang granuliert wird, dass die so erhaltenen Granülen in einem Fließbetttrockner getrocknet werden, dass die so erhaltenen Granülen auf eine Korngröße von 20 mesh gemahlen werden, dass das so erhaltene körnige Bindemittel mit Tolperison-Hydrochlorid als 50/50-Racemat in einem Hochgeschwindigkeitsmischer/Granulierer 2 min lang gemischt, unter Mischen mit einer Lösung von Ethylcellulose in Ethanol versetzt und anschließend 2 min lang granuliert wird und dass die so erhaltenen Granülen in einem Fließbetttrockner getrocknet, auf eine Korngröße von 20 mesh gemahlen und dann mit einem Tablettierhilfsmittel 5 min lang gemischt und zu Tabletten gepresst werden.

8. Verfahren zum Herstellen einer pharmazeutischen zubereitung zur oralen verabreichung, die Tolperison enthält, wobei die Zubereitung zur verzögerten Freisetzung des Wirkstoffes Tolperison zubereitet ist, **dadurch gekennzeichnet, dass** ein Bindemittel hergestellt wird, indem Xanthan-Gummi, Johannisbrot-Gummi, Calciumsulfat und Dextrose in einem Hochgeschwindigkeitsmischer/Granulierer 2 min lang trocken vermengt werden, dass nach Zugabe von Wasser 2 min lang granuliert wird, dass die so erhaltenen Granülen in einem Fließbetttrockner getrocknet werden, dass die so erhaltenen Granülen auf eine Korngröße von 20 mesh gemahlen werden, dass das so erhaltene körnige Bindemittel mit Tolperison-Hydrochlorid als 90/10-Racemat mit überwiegendem Anteil an (-)-Isomeren in einem Hochgeschwindigkeitsmischer/Granulierer 2 min lang gemischt, unter Mischen mit einer Lösung von Ethylcellulose in Ethanol versetzt und anschließend 2 min lang granuliert wird und dass die so erhaltenen Granülen in einem Fließbetttrockner getrocknet, auf eine Korngröße von 20 mesh gemahlen und dann mit einem Tablettier-Kilfsmittel 5 min lang gemischt und zu Tabletten gepresst - werden .

9. Verfahren nach Anspruch 7 oder B, **dadurch gekennzeichnet, dass** das Bindemittel aus einem Gemenge aus 25 % Xanthan-Gummi, 25 % Johannisbrot-Gummi, 40 % Dextrose, 10 % Calciumsulfat und 10 % Wasser, das während des Granulierens zugegeben wird, hergestellt wird.

10. Verfahren nach Anspruch 7 oder 8, **dadurch gekennzeichnet, dass** als Tablettierhilfsmittel Natriumstearylfumarat verwendet wird.

11. Verfahren zum Herstellen einer pharmazeutischen Zubereitung zur oralen Verabreichung, die Tolperison oder ein Salz davon enthält, wobei die zubereitung zur verzögerten Freisetzung des Wirkstoffes Tolperison zubereitet ist, **dadurch gekennzeichnet, dass** Tolperison-Hydrochlorid als 50/50-Racemat, Lactose und Methylhydroxypropylcellulose in einem Wirbelschicht-Granulator unter Zugabe einer wässerigen, einen Farbstoff enthaltenden Lösung granuliert werden, dass die so erhaltenen Granülen zusammen mit Magnesiumstearat und feindispersem Siliziumdioxid durch ein Sieb mit einer Maschenweite von 1,25 mm gearbeitet und in einem Mischer homogenisiert werden und dass das so erhaltene Gemenge zu Tabletten verpresst wird.

12. Verfahren zum Herstellen einer pharmazeutischen Zubereitung zur oralen Verabreichung, die Tolperison oder ein Salz davon enthält, wobei die Zubereitung zur verzögerten Freisetzung des Wirkstoffes Tolperison zubereitet ist, **dadurch gekennzeichnet, dass** Tolperison-Hydrochlorid als 65/35-Racemat mit überwiegendem Anteil an (-)-Isomeren, Lactose und Methylhydroxypropylcellulose in einem Wirbelschicht-Granulator unter Zugabe einer wässerigen, einen Farbstoff enthaltenden Lösung granuliert werden, dass die so erhaltenen Granülen zusammen mit Magnesiumstearat und feindispersem Siliziumdioxid durch ein Sieb mit einer Maschenweite von 1,25 mm gearbeitet und in einem Mischer homogenisiert werden und dass das so erhaltene Gemenge zu Tabletten verpresst wird.

13. Verfahren zum Herstellen einer pharmazeutischen Zubereitung zur oralen Verabreichung, die Tolperison oder ein Salz davon enthält, wobei die Zubereitung zur verzögerten Freisetzung des Wirkstoffes Tolperison zubereitet ist, **dadurch gekennzeichnet, dass** Tolperison-Hydrochlorid als 90/10-Racemat mit überwiegendem Anteil an (-)-Isomeren, Lactose und Methylhydroxypropylcellulose in einem Wirbelschicht-Granulator unter Zugabe einer wässerigen, einen Farbstoff enthaltenden Lösung granuliert werden, dass die so erhaltenen Granülen zusammen mit Magnesiumstearat und feindispersem Siliziumdioxid durch ein Sieb mit einer Maschenweite von 1,25 mm gearbeitet und in einem Mischer homogenisiert werden und dass das so erhaltene Gemenge zu Tabletten verpresst wird.

## Claims

1. Method for producing a pharmaceutical preparation for oral administration, which contains Tolperison or a salt thereof, the preparation being prepared for delayed release of the active ingredient Tolperison, **characterised in that** crystalline Tolperison hydrochloride as a 50/50 racemate is coated in a coating column, which is operated with an airflow, with a mixture of methanol and a solution of ethyl cellulose and hydroxypropyl cellulose in chloroform, the solution being sprayed at a temperature of 60°C at the inlet into the coating column with 2.5 bar pressure and at a rate of 60 ml/min, and **in that** the Tolperison crystals provided with the polymer coating are dried.

2. Method for producing a pharmaceutical preparation for oral administration, which contains Tolperison or a salt thereof, the preparation being prepared for delayed release of the active ingredient Tolperison, **characterised in that** Tolperison as a 90/10 racemate with a predominant proportion of (-) isomer is coated in a coating column, which is operated with an airflow, with a mixture of methanol and a solution of ethyl cellulose and hydroxypropyl cellulose in chloroform, the solution being sprayed at a temperature of 60°C at the inlet into the coating column with 2.5 bar pressure and at a rate of 60 ml/min, and **in that** the Tolperison crystals provided with the polymer coating are dried.

3. Method for producing a pharmaceutical preparation for oral administration, which contains Tolperison or a salt thereof, the preparation being prepared for delayed release of the active ingredient Tolperison, **characterised in that**, in an aqueous solution of Tolperison which is saturated with Tolperison, microcapsules which contain Tolperison are suspended.

4. Method according to claim 3, **characterised in that** the dissolved Tolperison contained in the microcapsules is present as a SO/50 racemate.

5. Method according to claim 3, **characterised in that** the dissolved Tolperison contained in the microcapsules is present as an 80/20 racemate with a predominant proportion of (-) isomer.

6. Method according to claim 3, **characterised in that** the dissolved Tolperison contained in microcapsules is present as a 70/30 racemate with a predominant proportion of (-) isomer.

7. Method for producing a pharmaceutical preparation for oral administration, which contains Tolperison, the preparation being prepared for delayed release of the active ingredient Tolperison, **characterised in that** a binder is produced by dry-mixing xanthene gum, carob gum, calcium sulphate and dextrose in a high speed mixer/granulator for 2 minutes, **in that** granulation is continued for 2 minutes after addition of water, **in that** the thus obtained granules are dried in a fluid-bed dryer, **in that** the thus obtained granules are ground to a grain size of 20 mesh, **in that** the thus obtained granular binder is mixed with Tolperison hydrochloride as a 50/50 racemate in a high speed mixer/granulator for 2 minutes, is mixed by mixing with a solution of ethyl cellulose in ethanol and subsequently is granulated for 2 minutes, and **in that** the thus obtained granules are dried in a fluid-bed dryer, ground to a grain size of 20 mesh and then mixed with a tabletting aid for 5 minutes and compressed into tablets.

8. Method for producing a pharmaceutical preparation for oral administration, which contains Tolperison, the preparation being prepared for delayed release of the active ingredient Tolperison, **characterised in that** a binder is produced by dry-mixing xanthene gum, carob gum, calcium sulphate and dextrose in a high speed mixer/granulator for 2 minutes, **in that** granulation is continued for 2 minutes after addition of water, **in that** the thus obtained granules are dried in a fluid-bed dryer, **in that** the thus obtained granules are ground to a grain size of 20 mesh, **in that** the thus obtained granular binder is mixed with Tolperison hydrochloride as a 90/10 racemate with a predominant proportion of (-) isomer in a high speed mixer/granulator for 2 minutes, is mixed by mixing with a solution of ethyl cellulose in ethanol and subsequently is granulated for 2 minutes, and **in that** the thus obtained granules are dried in a fluid-bed dryer, ground to a grain size of 20 mesh and then mixed with a tabletting aid for 5 minutes and compressed into tablets.

9. Method according to claim 7 or 8, **characterised in that** the binder is produced from a mixture of 25% xanthene gum, 25% carob gum, 40% dextrose, 10% calcium sulphate and 10% water which is added during granulation.

10. Method according to claim 7 or 8, **characterised in that** sodium stearyl fumarate is used as tabletting aid.

11. Method for producing a pharmaceutical preparation for oral administration, which contains Tolperison or a salt thereof, the preparation being prepared for delayed release of the active ingredient Tolperison, **characterised in that** Tolperison hydrochloride as a 50/50 racemate, lactose and methylhydroxypropyl cellulose are granulated in a fluid bed granulator with the addition of an aqueous solution which contains a colourant, **in that** the thus obtained granules are worked together with magnesium stearate and finely dispersed silicon dioxide through a screen with a mesh width of 1.25 mm and are homogenised in a mixer, and **in that** the thus obtained mixture is compressed into tablets.

12. Method for producing a pharmaceutical preparation for oral administration, which contains Tolperison or a salt thereof, the preparation being prepared for delayed release of the active ingredient Tolperison, **characterised in that** Tolperison hydrochloride as a 65/35 racemate with a predominant proportion of (-) isomer, lactose and methylhydroxypropyl cellulose are granulated in a fluid-bed granulator with the addition of an aqueous solution which contains a colourant, **in that** the thus obtained granules are worked together with magnesium stearate and finely dispersed silicon dioxide through a screen with a mesh width of 1.25 mm and are homogenised in a mixer, and **in that** the thus obtained mixture is compressed into tablets.

13. Method for producing a pharmaceutical preparation for oral administration, which contains Tolperison or a salt thereof, the preparation being prepared for delayed release of the active ingredient Tolperison, **characterised in that** Tolperison hydrochloride as a 90/10 racemate with a predominant proportion of (-) isomer, lactose and methylhydroxypropyl cellulose are granulated in a fluid-bed granulator with the addition of an aqueous solution which contains a colourant, **in that** the thus obtained granules are worked together with magnesium stearate and finely dispersed silicon dioxide through a screen with a mesh width of 1.25 mm and are homogenised in a mixer, and **in that** the thus obtained mixture is compressed into tablets.

## Revendications

1. Procédé de préparation d'une composition pharmaceutique destinée à l'administration par voie orale, qui contient de la tolpérisone ou un sel de celle-ci, la composition étant préparée de façon à permettre une libération retardée du principe actif tolpérisone, **caractérisé en ce que** le chlorhydrate de tolpérisone cristallin est enrobé, sous forme de racémate 50/50, dans une colonne d'enrobage fonctionnant au moyen d'un courant d'air, avec un mélange de méthanol et d'une solution d'éthylcellulose et d'hydroxypropylcellulose dans du chloroforme, la solution étant injectée dans la colonne d'enrobage à une température de 60°C à l'entrée de la colonne, sous une pression de 2,5 bar et à une vitesse de 60 ml/min et **en ce que** les cristaux de tolpérisone enrobés avec le polymère sont séchés.

2. Procédé de préparation d'une composition pharmaceutique destinée à l'administration par voie orale, qui contient de la tolpérisone ou un sel de celle-ci, la composition étant préparée de façon à permettre une libération retardée du principe actif tolpérisone, **caractérisé en ce que** la tolpérisone est enrobée, sous forme de racémate 90/10 dans lequel l'isomère (-) est présent dans une proportion prépondérante, dans une colonne d'enrobage fonctionnant au moyen d'un courant d'air, avec un mélange de méthanol et d'une solution d'éthylcellulose et d'hydroxypropylcellulose dans du chloroforme, la solution étant injectée dans la colonne d'enrobage à une température de 60°C à l'entrée de la colonne, sous une pression de 2,5 bar et à une vitesse de 60 ml/min et que les cristaux de tolpérisone enrobés avec le polymère sont séchés.

3. Procédé de préparation d'une composition pharmaceutique destinée à l'administration par voie orale, qui contient de la tolpérisone ou un sel de celle-ci, la composition étant préparée de façon à permettre une libération retardée du principe actif tolpérisone, **caractérisé en ce que** des microcapsules contenant de la tolpérisone sont mises en suspension dans une solution aqueuse de tolpérisone qui est saturée en tolpérisone.

4. Procédé selon la revendication 3, **caractérisé en ce que** la tolpérisone dissoute et celle contenue dans les microcapsules sont présentes sous forme de racémate 50/50.

5. Procédé selon la revendication 3, **caractérisé en ce que** la tolpérisone dissoute et celle contenue dans les microcapsules sont présentes sous forme de racémate 80/20 dans lequel l'isomère (-) est présent dans une proportion prépondérante.

6. Procédé selon la revendication 3, **caractérisé en ce que** la tolpérisone dissoute et celle contenue dans les microcapsules sont présentes sous forme de racémate 70/30 dans lequel l'isomère (-) est présent dans une proportion prépondérante.

7. Procédé de préparation d'une composition pharmaceutique destinée à l'administration par voie orale, qui contient de la tolpérisone, la composition étant préparée de façon à permettre une libération retardée du principe actif tolpérisone, **caractérisé en ce qu'**un liant est préparé par le fait que de la gomme xanthane, de la gomme de caroubier, du sulfate de calcium et du dextrose sont mélangés à sec dans un mélangeur/granulateur à grande vitesse pendant 2 minutes, **en ce qu'**après addition d'eau, ils sont soumis à une granulation pendant 2 minutes, **en ce que** les granules ainsi obtenus sont séchés dans un sécheur à lit fluidisé, **en ce que** les granules ainsi obtenus sont broyés à une grosseur de grain de 20 mesh, **en ce que** le liant granulaire ainsi obtenu est mélangé avec du chlorhydrate de tolpérisone sous forme de racémate 50/50 dans un mélangeur/granulateur à grande vitesse pendant 2 minutes tandis qu'une solution d'éthylcellulose dans de l'éthanol est introduite, sous agitation, dans le mélange obtenu et qu'ensuite, ce mélange est soumis à une granulation pendant 2 minutes, et **en ce que** les granules ainsi obtenus sont séchés dans un sécheur à lit fluidisé, broyés à une grosseur de grain de 20 mesh puis mélangés à un adjuvant de préparation de comprimés pendant 5 minutes et compressés en comprimés.

8. Procédé de préparation d'une composition pharmaceutique destinée à l'administration par voie orale, qui contient de la tolpérisone, la composition étant préparée de façon à permettre une libération retardée du principe actif tolpérisone, **caractérisé en ce qu'**un liant est préparé par le fait que de la gomme xanthane, de la gomme de caroubier, du sulfate de calcium et du dextrose sont mélangés à sec dans un mélangeur/granulateur à grande vitesse pendant 2 minutes, **en ce qu'**après addition d'eau, ils sont soumis à une granulation pendant 2 minutes, **en ce que** les granules ainsi obtenus sont séchés dans un sécheur à lit fluidisé, **en ce que** les granules ainsi obtenus sont broyés à une grosseur de grain de 20 mesh, **en ce que** le liant granulaire ainsi obtenu est mélangé avec du chlorhydrate de tolpérisone sous forme de racémate 90/10 dans lequel l'isomère (-) est présent dans une proportion prépondérante, dans un mélangeur/granulateur à grande vitesse pendant 2 minutes, tandis qu'une solution d'éthylcellulose dans de l'éthanol est introduite, sous agitation, dans le mélange obtenu et qu'ensuite, ce mélange est soumis à une granulation pendant 2 minutes, et **en ce que** les granules ainsi obtenus sont séchés dans un sécheur à lit fluidisé, broyés à une grosseur de grain de 20 mesh puis mélangés à un adjuvant de préparation de comprimés pendant 5 minutes et compressés en comprimés.

9. Procédé selon la revendication 7 ou 8, **caractérisé en ce que** le liant est obtenu à partir d'un mélange de 25 % de gomme xanthane, de 25 % de gomme de caroubier, de 40 % de dextrose, de 10 % de sulfate de calcium et de 10 % d'eau qui est ajoutée pendant la granulation.

10. Procédé selon la revendication 7 ou 8, **caractérisé en ce que** comme adjuvant de préparation de comprimés, on utilise du fumarate de stéaryle sodique.

11. Procédé de préparation d'une composition pharmaceutique destinée à l'administration par voie orale, qui contient de la tolpérisone ou un sel de celle-ci, la composition étant préparée de façon à permettre une libération retardée du principe actif tolpérisone, **caractérisé en ce que** du chlorhydrate de tolpérisone sous forme de racémate 50/50, du lactose et de la méthylhydroxypropylcellulose sont soumis à une granulation dans un granulateur à lit fluidisé avec introduction d'une solution aqueuse contenant un colorant, **en ce que** les granules ainsi obtenus sont passés, conjointement avec du stéarate de magnésium et du dioxyde de silicium finement dispersé, à travers un crible ayant une ouverture de maille de 1,25 mm, et ils sont homogénéisés dans un mélangeur et **en ce que** le mélange ainsi obtenu est compressé en comprimés.

12. Procédé de préparation d'une composition pharmaceutique destinée à l'administration par voie orale, qui contient de la tolpérisone ou un sel de celle-ci, la composition étant préparée de façon à permettre une libération retardée du principe actif tolpérisone, **caractérisé en ce que** du chlorhydrate de tolpérisone sous forme de racémate 65/35, dans lequel l'isomère (-) est présent dans une proportion prépondérante, du lactose et de la méthylhydroxypropylcellulose sont soumis à une granulation dans un granulateur à lit fluidisé avec introduction d'une solution aqueuse contenant un colorant, **en ce que** les granules ainsi obtenus sont passés, conjointement avec du stéarate de magnésium et du dioxyde de silicium finement dispersé, à travers un crible ayant une ouverture de maille de 1,25 mm, et ils sont homogénéisés dans un mélangeur et **en ce que** le mélange ainsi obtenu est compressé en comprimés.

13. Procédé de préparation d'une composition pharmaceutique destinée à l'administration par voie orale, qui contient de la tolpérisone ou un sel de celle-ci, la composition étant préparée de façon à permettre une libération retardée du principe actif tolpérisone, **caractérisé en ce que** du chlorhydrate de tolpérisone sous forme de racémate 90/10, dans lequel l'isomère (-) est présent dans une proportion prépondérante, du lactose et de la méthylhydroxypropylcellulose sont soumis à une granulation dans un granulateur à lit fluidisé avec introduction d'une solution aqueuse contenant un colorant, **en ce que** les granules ainsi obtenus sont passés, conjointement avec du stéarate de magnésium et du dioxyde de silicium finement dispersé, à travers un crible ayant une ouverture de maille de 1,25 mm et ils sont homogénéisés dans un mélangeur et **en ce que** le mélange ainsi obtenu est compressé en comprimés.
